# EUROPEAN PATENT SPECIFICATION

(11) **EP 2 282 989 B1**
(45) Date of publication and mention of the grant of the patent: **12.10.2011**
(21) Application number: 09753876.3
(22) Date of filing: 26.05.2009
(51) Int. Cl.: C07C 241/02, C07C 243/14

(54) **PROCESS FOR PREPARING 3-(2,2-DIMETHYLHYDRAZINO)METHYLPROPIONATE**
VERFAHREN ZUR HERSTELLUNG VON 3-(2,2-DIMETHYLHYDRAZIN)METHYLPROPIONAT
PROCÉDÉ POUR LA PRÉPARATION DU PROPIONATE 3-(2,2-DIMÉTHYLHYDRAZINIUM)

(30) Priority: 26.05.2008 EP 08156903; 18.09.2008 EP 08164565
(43) Date of publication of application: 16.02.2011
(73) Proprietor: Grindeks, a joint stock company, 1057 Riga (LV)
(72) Inventor: ZICANE, Daina, 1048 Riga (LV); TURKS, Maris, 1048 Riga (LV)
(86) International application number: PCT/EP2009/056370
(87) International publication number: WO 2009/144221

(56) References cited:
- DATABASE CA [Online] CHEMICAL ABSTRACTS SERVICE, COLUMBUS, OHIO, US; 1965, XP002540342 retrieved from STN Database accession no. 64:3589 & KOST, A. N. ET AL: "Solvent effect and steric factors in the Michael reaction" ZHURNAL ORGANICHESKOI KHIMII , 1(8), 1341-8 CODEN: ZORKAE; ISSN: 0514-7492, 1965,
- GILLER S A ET AL: "Derivatives of 1-H-Aziridine-2-Carboxylic acid", CHEMISTRY OF HETEROCYCLIC COMPOUNDS, KLUWER ACADEMIC PUBLISHERS-CONSULTANTS BUREAU, NE, vol. 11, 1 January 1975 (1975-01-01), pages 1378-1382, XP002490140, ISSN: 1573-8353, DOI: DOI:10.1007/BF00764530
- ZAPEVALOVA: RUSSIAN CHEMICAL BULLETIN, vol. 15, no. 12, 1966, pages 2127-2130,

## Description

### Technical Field

The present invention relates to an improved process for preparation of 3-(2,2-dimethylhydrazino)methylpropionate.

### Background Art

A number of processes for the preparation of 3-(2,2,2-trimethylhydrazinium)propionate dihydrate is known.

The first process for preparation 3-(2,2,2-trimethylhydrazinium)propionate dihydrate is disclosed in WO 80/01068 A (INST ORGANICHESKOGO SINTEZA) 1980.05.29.. The process starts with methyl 3-(2,2-dimethylhydrazino)propionate is treated with a methyl halide or dimethylsulphate to give the appropriate trimethylhydrazinium salt, which is transferred to 3-(2,2,2-trimethylhydrazinium)propionate by Amberlite IRA-400 (OH form). After crystallization from ethanol the inner salt is obtained as a dihydrate.

This process disclosed in above patent can be considered as a common process for 3-(2,2,2-trimethylhydrazinium)propionate dihydrate preparation.

There are several processes for obtaining 3-(2,2,2-trimethylhydrazinium) propionate dihydrate, but most of them are concentrated to the avoid use of electrodialysis, which include many disadvantages: strongly basic ion exchangers are unstable and undergo decomposition and oxidation during processing; they withstand only a limited number of regeneration cycles; large quantities or solvents, acids and bases as well as deionised water are needed to regenerate the resins; low ion exchange capacity and therefore high production costs of 3-(2,2,2-trimethylhydrazinium)propionate dihydrate by this process are typical. However at the same time, process of obtaining intermediates in high quality for producing 3-(2,2,2-trimethylhydrazinium)propionate as well is very important.

One of such intermediates is 3-(2,2-dimethylhydrazino)methylpropionate. Common process to produce this intermediate is reacting 1,1-dimethylhydrazine with methyl acrylate.

Reaction between 1,1-dimethylhydrazine with methyl acrylate which lead either to a cyclic 1,1-dimethyl-2-pyrazolinium-3-olate or to the linear product of the addition of DMH at the double bond of the ester is described in ZAPEVALOVA, N. P. , et al. Reactions of 1,1-disubstituted hydrazines with derivatives of α,β-unsatur ated acids Communication 4. Reactions of 1,1-dimethylhydrazine with α,β-unsaturated esters. Russian Chemical Bulletin. 1966, vol.15, no.12, p.2127-2130. Another reaction of 1,1-dimethylhydrazine with acrylic acid derivatives gave a number of 1,1-dimethyl-2-(2-substituted ethyl)hydrazines which described in GILLER, S.A., et al. Derivatives of 1-H-aziridine-2-carboxylic acid. Chemistry of Heterocyclic Compounds. 1975, vol.11, no.12, p.1378-1382. KOST in Zhurnal Organicheskoi Khimii (1965), 1 (8), 1341-8 discloses the synthesis of 3-(2,2-dimethylhydrazino)methylpropionate starting from dimethylhydrazine and methyl acrylate in ethanol in the presence of hydroquinone.

### Disclosure of Invention

Under standard conditions, by reaction of 1,1-dimethylhydrazine with methyl acrylate, in the absence of an antioxidant several side products IV-VII were observed. Thus, dimethylamine (IV) forms in the oxidation of 1,1-dimethylhydrazine (I). The former can react with methyl acrylate (II) and formed wanted side product methyl 3-(dimethylamino)propanoate (V). Similary, product 3-(2,2-dimethylhydrazino)methylpropionate (III) is sensitive to air and slowly oxidizes to methyl 2-(2,2-dimethylhydrazono)acetate (VI). As well that antioxidant suppresses double addition which can form unwanted dimmer dimethyl 3,3'-(2,2-dimethylhydrazine-1,1-diyl)dipropanoate (VII), see Table 1 and Table 2.

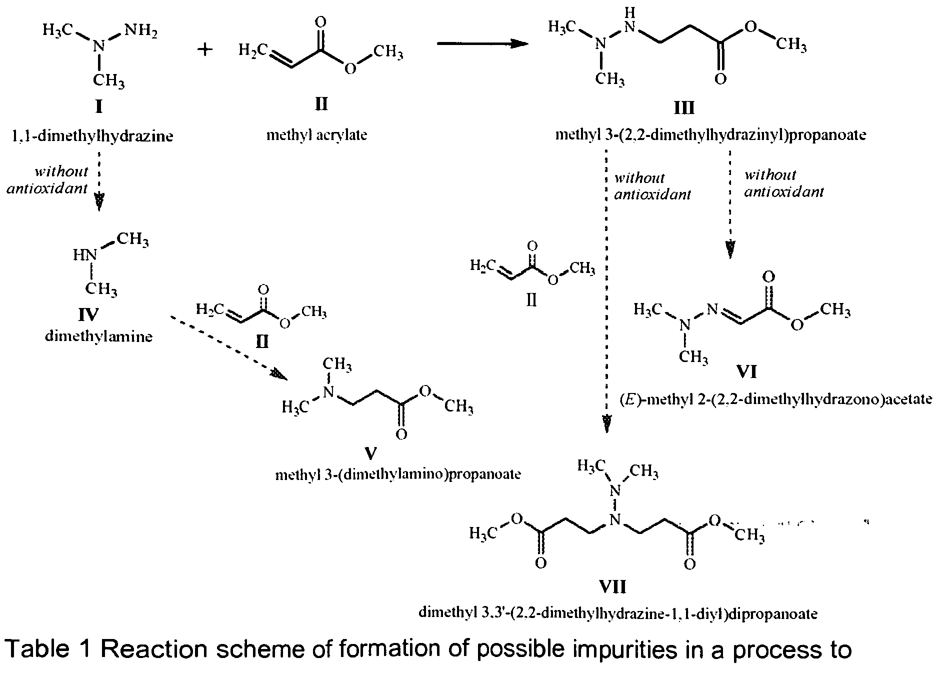

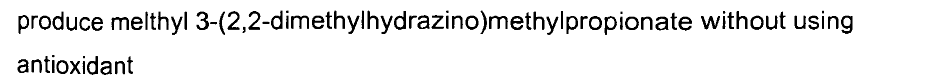

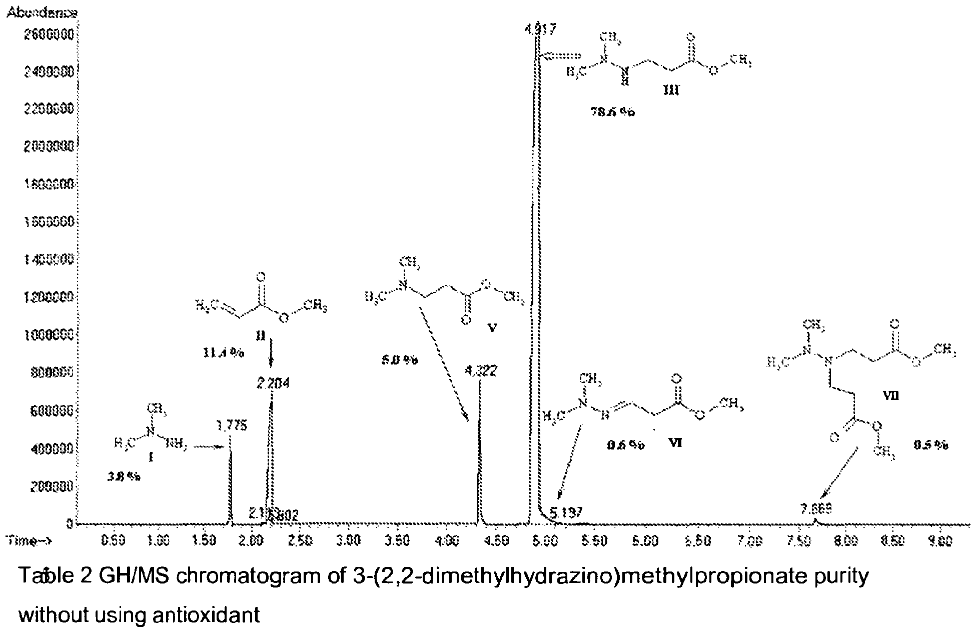

Surprisingly, we found out what the above objective is achieved according to present invention by reaction of 1,1-dimethylhydrazine (I) with methyl acrylate (II) by using citric acid as antioxidant, thus preparing 3-(2,2-dimethylhydrazino)methylpropionate (III).

In GB950622 A (CELANESE CORP) 1964.02.26 is disclosed what antioxidant, such as hydroquinone, can be used in order to stabilize methyl acrylate and to prevent its polymerization.

However, the present invention deals with stabilization of 1,1-dimethylhydrazine and 3-(2,2-dimethylhydrazino)methylpropionate, besides yield and purity of 3-(2,2-dimethylhydrazino)methylpropionate was increased significantly by using citric acid as antioxidant. acid is used as antioxidant and catalyzed first step process and reducing formation of impurities, such us IV-VII, which form by oxidation of 3-(2,2-dimethylhydrazino)methylpropionate as well remains of starting products 1,1-dimethylhydrazine and methyl acrylate, see Table 3 and Table 4.

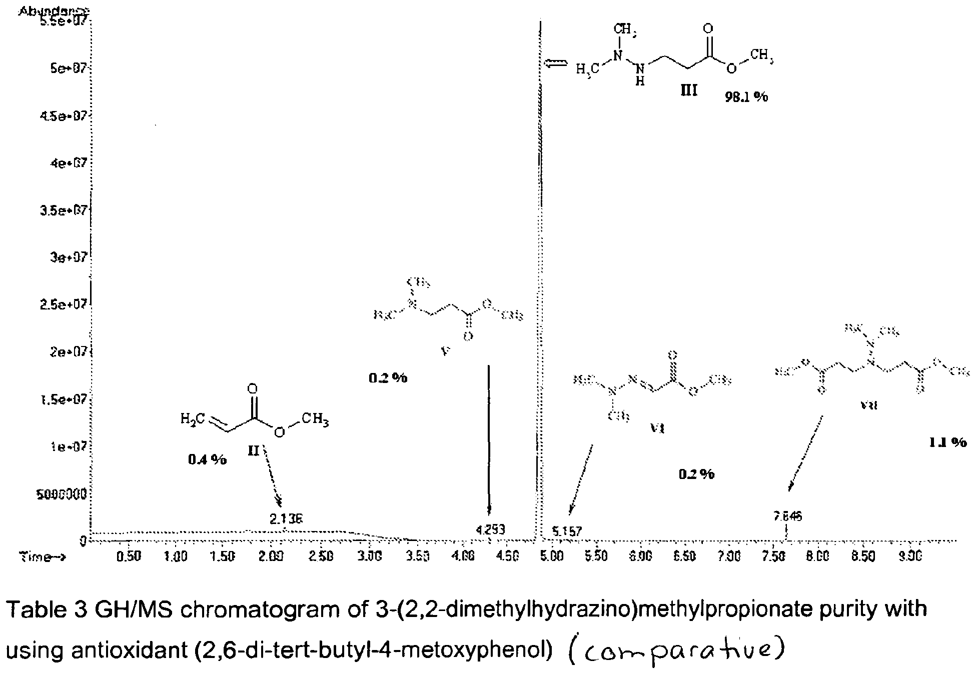

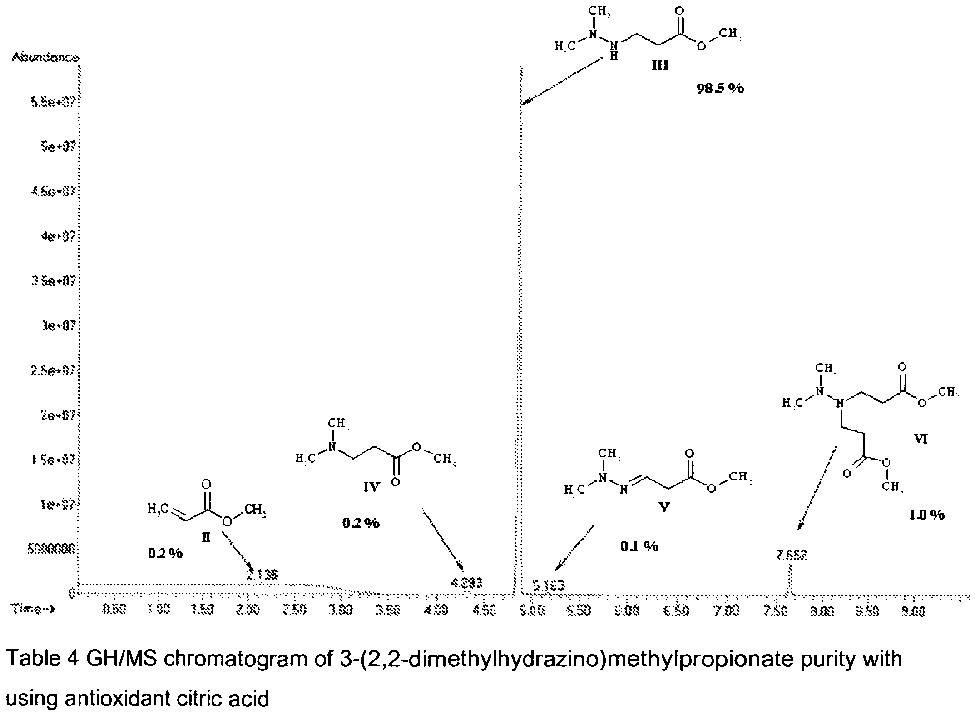

Thus, the subject matter of the present invention is a process for preparing a compound 3-(2,2-dimethylhydrazino)methylpropionate having formula III, by reaction of compound I with methyl acrylate of formula II to yield 3-(2,2-dimethylhydrazino)methylpropionate of formula III by using citric acid antioxidant which acts as catalyst.

### Best Mode for Carrying Out the Invention

The present invention will be described in more detail by referring to the following examples.

### Example 1 (Comparative)

Preparation of 3-(2,2-dimethylhydrazino)methylpropionate by antioxidant 1,1-Dimethylhydrazine was added to stirred antioxidant in 3-neck round bottom flask fitted with mechanical stirrer, a dropping funnel and a reflux condenser.

The reaction mixture was stirred and heated to 50±5°C, at which point methyl acrylate was added. Thereafter the reaction mixture was heated at 80±85°C until formation 3-(2,2-dimethylhydrazino)methylpropionate (controlled by GM-MS). Great list of antioxidants were used, but surprisingly just two of them are the most suitable for used as an antioxidant, by obtaining 3-(2,2-dimethylhydrazino)methylpropionate in high yields, see Table 5.

| **Antioxidant** | **Time, h** | **Yield of 3-(2,2-dimethylhydrazino) methylpropionate (%)** |
|---|---|---|
| Hydroquinone | 1 | 87 |
| | 2 | 90 |
| **2,5-di***-****tert-*butylhydroquinone** | 1 | 86,2 |
| | 2 | **95** |
| 2,6-di-*tert*-butyl-4-methylphenol | 1 | 85,4 |
| | 2 | 87 |
| 6-*O*-palmitoyl ascorbic acid | 1 | 87,1 |
| | 2 | 89 |
| propyl gallate | 1 | 83,3 |
| | 2 | 88 |
| butylated hydroxyanisole | 1 | 91,7 |
| | 2 | 91 |
| n-dodecyl gallate | 1 | 85,5 |
| | 2 | 90 |
| L(+)-ascorbic acid | 1 | 85 |
| | 2 | 88,5 |
| L-ascorbic acid sodium salt | 1 | 87 |
| | 2 | 89 |
| D-isoascorbic acid | 1 | 86,5 |
| | 2 | 88,5 |
| Citric acid | 1 | 89,5 |
| | 2 | **97.3** |

### Example 2 (Comparative)

### Preparation of 3-(2,2-dimethylhydrazino)methylpropionate by using 2-tert-butylhydroquinone as antioxidant

1,1-Dimethylhydrazine (35.0g, 0.63 mol) was added to stirred 2-tert-butylhydroquinone (0.18 g, 1.1 mmol) in 3-neck round bottom flask fitted with mechanical stirrer, a dropping funnel and a reflux condenser.

The reaction mixture was stirred and heated to 50±5°C, at which point methyl acrylate (51.6g, 0.6mol) was added. Thereafter the reaction mixture was heated at 80±85°C for 2.5 hours until formation 3-(2,2-dimethylhydrazino)methylpropionate (controlled by GM-MS). Yield 95%.

### Example 3

### Preparation of 3-(2,2-dimethylhydrazino)methylpropionate by using citric acid as antioxidant

1,1-Dimethylhydrazine (37.9g, 0.63 mol) was added to stirred citric acid (0.19 g, 1.1 mmol) in 3-neck round bottom flask fitted with mechanical stirrer, a dropping funnel and a reflux condenser.

The reaction mixture was stirred and heated to 50±5°C, at which point methyl acrylate (47.9g, 0.6 mol) was added. Thereafter the reaction mixture was heated at 80±85°C until formation 3-(2,2-dimethylhydrazino)methylpropionate (controlled by GM-MS). Yield 97.3%.

## Claims

1. A process for preparing 3-(2,2-dimethylhydrazino)methylpropionate of formula **III** 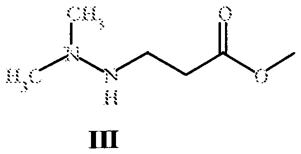 by reaction of 1,1-dimethylhydrazine with methyl acrylate in presence of citric acid.

## Patentansprüche

1. Ein Verfahren zur Herstellung von 3 - (2,2-dimethylhydrazino) methylpropionat der Formel **III** 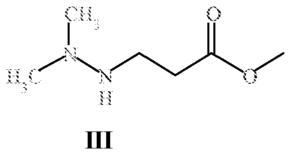 durch Umsetzung von 1,1-Dimethylhydrazin mit Methylacrylat in Gegenwart von Zitronensäure.

## Revendications

1. La procédure de la préparation de 3-(2,2- dimethylhydrasine) methyl propionate de la formule III 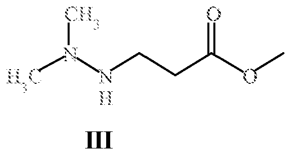 par réaction de 1,1- dimethylhydrasine avec methylacrylate en présence d'acide citrique.
